# EUROPEAN PATENT SPECIFICATION

(11) **EP 1 051 494 B1**
(45) Date of publication and mention of the grant of the patent: **29.04.2009**
(21) Application number: 99903498.6
(22) Date of filing: 29.01.1999
(51) Int. Cl.: C12N 15/29, A61K 39/35, C07K 14/415, A01H 5/00, A01K 67/027

(54) **METHODS AND REAGENTS FOR DECREASING ALLERGIC REACTIONS**
VERFAHREN UND REAGENZIEN ZUR VERMINDERUNG DER KLINISCHEN REAKTION ZUR ALLERGIE
PROCEDES ET REACTIFS PERMETTANT DE DIMINUER LES REACTIONS ALLERGIQUES

(30) Priority: 31.01.1998 US 73283 P; 13.02.1998 US 74590 P; 13.02.1998 US 74624 P; 13.02.1998 US 74633 P; 27.08.1998 US 141220
(43) Date of publication of application: 15.11.2000
(73) Proprietor: THE UNIVERSITY OF ARKANSAS, Little Rock, AR 72207-3608 (US); Mount Sinai School of Medicine of New York University, New York, NY 10029-6574 (US)
(72) Inventor: BANNON, Gary, A., Little Rock, AR 72211 (US); BURKS, A., Wesley, Jr., Little Rock, AR 72207 (US); SAMPSON, Hugh, A., Larchmont, NY 10538 (US); Gael Cocknell,The University of Arkansas, Arkansas, 72207 (US); Ricki M Helm,The University of Arkansas, Arkansas, 72207 (US); Wina E King, The University of Arkansas, Arkansas, 72207 (US); J Seven Stanley, The University of Aarkansas, Arkansas, 72207 (US); David S Shin, The University of Arkansas, Arkansas, 72207 (US)
(74) Representative: Cornish, Kristina Victoria Joy
(86) International application number: PCT/US1999/002031
(87) International publication number: WO 1999/038978

(56) References cited:
- STANLEY JS ET AL: "Identification and mutational analysis of the immunodominant IgE binding epitopes of the major peanut allergen Ara h 2" ARCHIVES OF BIOCHEMISTRY AND BIOPHYSICS, vol. 342, no. 2, 15 June 1997, pages 244-253, XP002107202
- BURKS AW ET AL: "Mapping and mutational analysis of the IgE-binding epitopes on Ara h 1, a legume vicilin protein and a major allergen in peanut hypersensitivity " EUROPEAN JOURNAL OF BIOCHEMISTRY, vol. 245, no. 2, April 1997, pages 334-339, XP002107203
- BURKS AW ET AL: "Recombinant peanut allergen Ara h 1 expression and IgE binding in patients with peanut hypersensitivity" THE JOURNAL OF CLINICAL INVESTIGATION, vol. 96, no. 4, October 1995, pages 1715-1721, XP002107204

## Description

### Background of the Invention

The United States government has rights in this invention by virtue of grants from the National Institute of Health RO1-AI33596.

Allergic disease is a common health problem affecting humans and companion animals (mainly dogs and cats) alike. Allergies exist to foods, molds, grasses, trees, insects, pets, fleas, ticks and other substances present in the environment. It is estimated that up to 8% of young children and 2% of adults have allergic reactions just to foods alone. Some allergic reactions (especially those to foods and insects) can be so severe as to be life threatening. Problems in animals tend to be less severe, but very common. For example, many dogs and cats have allergies to flea saliva proteins, grasses, and other common substances present in the environment.

Allergy is manifested by the release of histamines and other mediators of inflammation by mast cells which are triggered into action when IgE antibodies bound to their receptors on the mast cell surface are cross linked by antigen. Other than avoidance, and drugs (e.g. antihistamines, decongestants, and steroids) that only treat symptoms and can have unfortunate side effects and often only provide temporary relief, the only currently medically accepted treatment for allergies is immunotherapy. Immunotherapy involves the repeated injection of allergen extracts, over a period of years, to desensitize a patient to the allergen. Unfortunately, traditional immunotherapy is time consuming, usually involving years of treatment, and often fails to achieve its goal of desensitizing the patient to the allergen. Furthermore, it is not the recommended treatment for food allergies, such as peanut allergies, due to the risk of anaphylaxis.

Noon (Noon, Lancet 1911; 1:1572-73) first introduced allergen injection immunotherapy in 1911, a practice based primarily on empiricism with non-standardized extracts of variable quality. More recently the introduction of standardized extracts has made it possible to increase the efficacy of immunotherapy, and double-blind placebo-controlled trials have demonstrated the efficacy of this form of therapy in allergic rhinitis, asthma and bee-sting hypersensitivity (BSAC Working Party, Clin. Exp. Allergy 1993; 23:1-44). However, increased risk of anaphylactic reactions has accompanied this increased efficacy. For example, initial trials of immunotherapy to food allergens has demonstrated an unacceptable safety:efficacy ratio (Oppenheimer et al. J Allergy Clin. Immun. 1992; 90:256-62; Sampson, J. Allergy Clin. Immun. 1992; 90:151-52; Nelson et al. J. Allergy Clin. Immun. 1996; 99:744-751). Results like these have prompted investigators to seek alternative forms of immunotherapy as well as to seek other forms of treatment.

Initial trials with allergen-non-specific anti-IgE antibodies to deplete the patient of allergen-specific IgE antibodies have shown early promise (Boulet, et al. 1997; 155:1835-1840; Fahy, et al. American J Respir. Crit. Care Med. 1997; 155:1828-1834; Demoly P. and Bousquet J. American J Resp. Crit. Care Med. 1997; 155:1825-1827). On the other hand, trials utilizing immunogenic peptides (representing T cell epitopes) have been disappointing (Norman, et al. J. Aller. Clin. Immunol. 1997; 99:S127). Another form of allergen-specific immunotherapy which utilizes injection of plasmid DNA (Raz et al. Proc. Nat. Acad. Sci. USA 1994; 91:9519-9523; Hz et al. Int. Immunol. 1996; 8:1405-1411) remains unproven.

There remains a need for a safe and efficacious therapy for allergies, especially those where traditional immunotherapy is ill advised due to risk to the patient or lack of efficacy. There is also a need for alternatives to therapies, for example, by creating foods, materials or substances that do not include the allergens that are most problematic, or which contain modified allergens which do not elicit the same reaction. While the technology to make genetically engineered plants and animals is at this point well established, useful modifications would require understanding how allergens can be modified so that they retain the essential functions for the plants' and animals' nutritional value, taste characteristics, etc., but no longer elicit as severe an allergic response.

It is therefore an object of the present invention to provide a method for decreasing the allergenicity of allergens by modifying the allergen itself and thus prevent binding of IgE.

It is a further object of the present invention to provide allergens that elicit fewer IgE mediated responses.

It is still another object of the present invention to provide a method to make genetically engineered plants and animals that elicit less of an allergic response than the naturally occurring organisms.

### Summary of the Invention

The present invention is as defined in the claims.

It has been determined that allergens, which are characterized by both humoral (IgG and IgE) and cellular (T cell) binding sites, can be made less allergenic by modifying the IgE binding sites. The IgE binding sites can be eliminated by altering as little as a single amino acid within each of the immunodominant epitopes of the protein to eliminate IgE binding. The method allows the protein to be altered as minimally as possible, (i.e. only within each of the immunodominant IgE-binding sites) while retaining the ability of the protein to activate T cells and, optionally, to bind IgG. Binding sites are identified using known techniques, such as by binding with antibodies in pooled sera obtained from individuals known to be immunoreactive with the allergen to be modified. Proteins that are modified to alter IgE binding are screened for binding with IgG and/or activation of T cells.

Peanut allergens (Ara h 1, Ara h 2, and Ara h 3) have been used in the examples to demonstrate alteration of IgE binding sites while retaining binding to IgG and activation of T cells. The critical amino acids within each of the IgE binding epitopes of the peanut protein that are important to immunoglobulin binding were determined. Substitution of even a single amino acid within each of the epitopes led to loss of IgE binding. Although the epitopes shared no common amino acid sequence motif, the hydrophobic residues located in the center of the epitope appeared to be most critical to IgE binding.

Standard techniques such as a skin test for wheal and flare formation can be used to assess decreased allergenicity of modified proteins, created as described in the examples. The modified allergens can also be tested for binding to IgG and proliferation of T cells, and modified allergens selected for optimal stimulation of T cells and binding IgG.

The immunotherapeutics can be delivered by standard techniques, using injection, by aerosol, sublingually, topically (including to a mucosal surface), and by gene therapy (for example, by injection of the gene encoding the immunotherapeutic into muscle or skin where it is transiently expressed for a time sufficient to induce tolerance).

This method and the criteria for identifying and altering allergens can be used to design useful proteins (including nucleotide molecules encoding the proteins) for use in immunotherapy, to make a vaccine and to genetically engineer organisms such as plants and animals which then produce proteins with less likelihood of eliciting an IgE response. Techniques for engineering plants and animals are well known. Based on the information obtained using the method described in the examples, one can engineer plants or animals to cause either site specific mutations in the gene encoding the protein(s) of interest, or to knock out the gene and then insert the gene encoding the modified protein.

### Brief Description of the Drawings

Figure 1 shows an example of how IgE binding epitopes were mapped to a specific amino acid sequence on the Ara h 1 allergen.
Figure 2 shows how IgE binding epitopes were mapped to a specific amino acid sequence on the Ara h 2 allergen.
Figure 3 shows how IgE binding epitopes were mapped to a specific amino acid sequence on the Ara h 3 allergen.
Figure 4 is a graph of the %IgE binding relative to wild type Ara h2 of modified Ara h 2 allergens.
Figure 5 shows the results of T-cell proliferation assays using the native and recombinant wild-type and modified Ara h 2 protein, compared to crude peanut as a control.

### Detailed Description of the Invention

### Definitions

The following definitions are used herein.

An antigen is a molecule that elicits production of antibody (a humoral response) or an antigen-specific reaction with T cells (a cellular response).

An allergen is a subset of antigens which elicits IgE production in addition to other isotypes of antibodies.

An allergic reaction is one that is IgE mediated with clinical symptoms primarily involving the cutaneous (uticaria, angiodema, pruritus), respiratory (wheezing, coughing, laryngeal edema, rhinorrhea, watery/itching eyes), gastrointestinal (vomiting, abdominal pain, diarrhea), and cardiovascular (if a systemic reaction occurs) systems.

An epitope is a binding site including an amino acid motif of between approximately six and fifteen amino acids which can be bound by either an immunoglobulin or recognized by a T cell receptor when presented by an antigen presenting cell in conjunction with the major histocompatibility complex (MHC). A linear epitope is one where the amino acids are recognized in the context of a simple linear sequence. A conformational epitope is one where the amino acids are recognized in the context of a particular three dimensional structure.

An immunodominant epitope is one which is bound by antibody in a large percentage of the sensitized population or where the titer of the antibody is high, relative to the percentage or titer of antibody reaction to other epitopes present in the same protein.

A decreased allergic reaction is characterized by a decrease in clinical symptoms following treatment of symptoms associated with exposure to an allergen, which can involve respiratory, gastrointestinal, skin, eyes, ears and mucosal surfaces in general.

An antigen presenting cell (an APC) is a cell which processes and presents peptides to T cells to elicit an antigen-specific response.

Immunostimulatory sequences are oligodeoxynucleotides of bacterial, viral or invertebrate origin that are taken-up by APCs and activate them to express certain membrane receptors (e.g., B7-1 and B7-2) and secrete various cytokines (e.g., IL-1, IL-6, IL-12, TNF). These oligodeoxynucleotides containing unmethylated CpG motifs cause brisk activation and when injected into animals in conjunction with antigen, appear to skew the immune response to a Th1-type response. See, for example, Yamamoto, et al., Microbiol. Immunol. 36, 983 (1992); Krieg, et al., Nature 374, 546-548 (1995); Pisetsky, Immunity 5, 303 (1996); and Zimmerman, et al., J. Immunol. 160, 3627-3630 (1998).

### I. Diagnostic and Therapeutic Reagents.

The first step in making the modified allergen is to identify IgE binding sites and/or immunodominant IgE binding sites. The second step is to mutate one or more of the IgE binding sites, including at a minimum each one of the immunodominant sites, or to react the allergen with a compound that selectively blocks binding to one or more of the IgE binding sites. The third step is to make sufficient amounts of the modified allergen for administration to persons or animals in need of tolerance to the allergen, where the modified allergen is administered in a dosage and for a time to induce tolerance, or for diagnostic purposes. The modified allergen can be administered by injection, or in some cases, by ingestion or inhalation.

### A. Allergens.

Many allergens are known that elicit allergic responses, which may range in severity from mildly irritating to life-threatening. Food allergies are mediated through the interaction of IgE to specific proteins contained within the food. Examples of common food allergens include proteins from peanuts, milk, grains such as wheat and barley, soybeans, eggs, fish, crustaceans, and mollusks. These account for greater than 90% of the food allergies (Taylor, Food Techn. 39,146-152 (1992). The IgE binding epitopes from the major allergens of cow milk (Ball, et al. (1994) Clin. Exp. Allergy, 24, 758-764), egg (Cooke, S.K. and Sampson, H.R. (1997) J. Immuno/., 159, 2026-2032), codfish (Aas, K., and Elsayed, S. (1975) Dev. Biol. Stand. 29, 90-98), hazel nut (Elsayed, et al. (1989) Int. Arch. Allergy Appl. Immunol. 89, 410-415), peanut (Burks et al., (1997) Eur. J. Biochemistry, 245:334-339; Stanley et al., (1997) Archives of Biochemistry and Biophysics, 342:244-253), soybean (Herein, et al. (1990) Int. Arch. Allergy Appl. Immunol. 92, 193-198) and shrimp (Shanty, et al. (1993) J. Immunol. 151, 5354-5363) have all been elucidated, as have others. Other allergens include proteins from insects such as flea, tick, mite, fire ant, cockroach, and bee as well as molds, dust, grasses, trees, weeds, and proteins from mammals including horses, dogs, cats, etc.

The majority of allergens discussed above elicit a reaction when ingested, inhaled, or injected. Allergens can also elicit a reaction based solely on contact with the skin. Latex is a well known example. Latex products are manufactured from a milky fluid derived from the rubber tree, *Hevea brasiliensis* and other processing chemicals. A number of the proteins in latex can cause a range of allergic reactions. Many products contain latex, such as medical supplies and personal protective equipment. Three types of reactions can occur in persons sensitive to latex: irritant contact dermatitis, and immediate systemic hypersensitivity. Additionally, the proteins responsible for the allergic reactions can fasten to the powder of latex gloves. This powder can be inhaled, causing exposure through the lungs. Proteins found in latex that interact with IgE antibodies were characterized by two-dimensional electrophoresis. Protein fractions of 56, 45, 30, 20, 14, and less than 6.5 kd were detected (Posch A. et al., (1997) J. Allergy Clin. Immunol. 99(3), 385-395 ). Acidic proteins in the 8-14 kd and 22 - 24 kd range that reacted with IgE antibodies were also identified (Posch A. et al., (1997) J. Allergy Clin. Immunol. 99(3), 385-395. The proteins prohevein and hevein, from hevea brasiliensis, are known to be major latex allergens and to interact with IgE (Alenius, H., et al., Clin. Exp. Allergy 25(7), 659-665; Chen Z., et al., (1997) J. Allergy Clin. Immunol. 99(3), 402-409). Most of the IgE binding domains have been shown to be in the hevein domain rather than the domain specific for prohevein (Chen Z., et al., (1997) J. Allergy Clin. Immunol. 99(3), 402-409). The main IgE-binding epitope of prohevein is thought to be in the N-terminal, 43 amino acid fragment (Alenius H., et al., (1996) J. Immunol. 156(4), 1618-1625). The hevein lectin family of proteins has been shown to have homology with potato lectin and snake venom disintegrins (platelet aggregation inhibitors) (Kielisqewski, M.L., et al., (1994) Plant J. 5(6), 849-861).

### B. Identification of IgE Binding Sites.

Allergens typically have both IgE and IgG binding sites and are recognized by T cells. The binding sites can be determined either by using phage display libraries to identify conformational epitopes (Eichler and Houghten, (1995) Molecular Medicine Today 1, 174-180; Jensen-Jarolim et al., (1997) J. Appl. Clin. Immunol. 101, 5153a) or by using defined peptides derived from the known amino acid sequence of an allergen (see examples below), or by binding of whole protein or protein fragments to antibodies, typically antibodies obtained from a pooled patient population known to be allergic to the allergen. It is desirable to modify allergens to diminish binding to IgE while retaining their ability to activate T cells and in some embodiments by not significantly altering or decreasing IgG binding capacity. This requires modification of each of the immunodominant IgE binding sites in the allergen.

A preferred modified allergen is one that can be used with a majority of patients having a particular allergy. Use of pooled sera from allergic patients allows determination of one or more immunodominant epitopes in the allergen. Once some or all of the IgE binding sites are known, it is possible to modify the gene encoding the allergen, using site directed mutagenesis by any of a number of techniques, to produce a modified allergen as described below, and thereby express modified allergens. It is also possible to react the allergen with a compound that achieves the same result as the selective mutation, by making the IgE binding sites inaccessible, but not preventing the modified allergen from activating T cells, and, in some embodiments, by not significantly altering or decreasing IgG binding.

Assays to assess an immunologic change after the administration of the modified allergen are known to those skilled in the art. Conventional assays include RAST (Sampson and Albergo, 1984), ELISAs (Burks, et al. 1986) immunoblotting (Burks, et al. 1988), and *in vivo* skin tests (Sampson and Albergo 1984). Objective clinical symptoms can be monitored before and after the administration of the modified allergen to determine any change in the clinical symptoms.

It may be of value to identify IgEs which interact with conformational rather than linear epitopes. Due to the complexity and heterogeneity of patient serum, it may be difficult to employ a standard immobilized allergen affinity-based approach to directly isolate these IgEs in quantities sufficient to permit their characterization. These problems can be avoided by isolating some or all of the IgEs which interact with conformational epitopes from a combinatorial IgE phage display library.

Steinberger et al. (Steinberger, P., Kraft D. and Valenta R. (1996) "Construction of a combinatorial IgE library from an allergic patient: Isolation and characterization of human IgE Fabs with specificity for the major Timothy Grass pollen antigen," Phl p. 5 J. Biol. Chem. 271, 10967-10972) prepared a combinatorial IgE phage display library from mRNA isolated from the peripheral blood mononuclear cells of a grass allergic patient. Allergen-specific IgEs were selected by panning filamentous phage expressing IgE Fabs on their surfaces against allergen immobilized on the wells of 96 well microtiter plates. The cDNAs were than isolated from allergen-binding phage and transformed into E coli for the production of large quantities of monoclonal, recombinant, allergen-specific IgE Fabs.

If native allergen or full length recombinant allergen is used in the panning step to isolate phage, then Fabs corresponding to IgEs specific for conformational epitopes should be included among the allergen-specific clones identified. By screening the individual recombinant IgE Fabs against denatured antigen or against the relevant linear epitopes identified for a given antigen, the subset of conformation-specific clones which do not bind to linear epitopes can be defined.

To determine whether the library screening has yielded a complete inventory of the allergen-specific IgEs present in patient serum, an immunocompetition assay can be performed. Pooled recombinant Fabs would be preincubated with immobilized allergen. After washing to remove unbound Fab, the immobilized allergen would then be incubated with patient serum. After washing to remove unbound serum proteins, an incubation with a reporter-coupled secondary antibody specific for IgE Fc domain would be performed. Detection of bound reporter would allow quantitation of the extent to which serum IgE was prevented from binding to allergen by recombinant Fab. Maximal, uncompeted serum IgE binding would be determined using allergen which had not been preincubated with Fab or had been incubated with nonsense Fab. If IgE binding persists in the face of competition from the complete set of allergen-specific IgE Fab clones, this experiment can be repeated using denatured antigen to determine whether the epitopes not represented among the cloned Fabs are linear or conformational.

### Production of Recombinant or Modified Allergens

A modified allergen will typically be made using recombinant techniques. Expression in a procaryotic or eucaryotic host including bacteria, yeast, and baculovirus-insect cell systems are typically used to produce large (mg) quantities of the modified allergen. It is also possible to make the allergen synthetically, if the allergen is not too large, for example, less than about 25-40 amino acids in length.

### Production of Transgenic Plants and Animals

Transgenic plants or animals expressing the modified allergens have two purposes. First, they can be used as a source of modified allergen for use in immunotherapy and second, appropriately modified plants or animals can be substituted for the original plant or animal, making immunotherapy unnecessary. Furthermore, it is possible that eating modified peanuts or cod fish, for example, could have either or both of two effects: (1) not imparting an allergic response on their own and (2) conferring protection from the unmodified source by acting as an immunotherapeutic agent for the unmodified source. Methods for engineering of plants and animals are well known and have been for a decade. For example, for plants see Day, (1996) Crit. Rev. Food Sci. & Nut. 36(S), 549-567, the teachings of which are incorporated herein. See also Fuchs and Astwood (1996) Food Tech. 83-88. Methods for making recombinant animals are also well established. See, for example, Colman, A" Production of therapeutic proteins in the milk of transgenic livestock" (1998) Biochem. Soc. Symp. 63, 141-147; Espanion and Niemann, (1996) DTW Dtxch Tierarztl Wochenschr 103(8-9), 320-328; and Colman, Am. J. Clin. Nutr. 63(4), 639S-6455S, the teachings of which are incorporated herein. One can also induce site specific changes using homologous recombination and/or triplex forming oligomers. See, for example, Rooney and Moore, (1995) Proc. Natl. Acad. Sci. USA 92, 2141-2149; Agrawal, et al., BioWorld Today, vol. 9, no. 41, p. 1 "Chimeriplasty - Gene Surgery, Not Gene Therapy - Fixes Flawed Genomic Sequences" David N. Leff.

### II. Diagnostic and Therapeutic Procedures Using Modified Allergens.

It is important to administer the modified allergen to an individual (human or animal) to decrease the clinical symptoms of allergic disease by using a method, dosage, and carrier which are effective. Allergen will typically be administered in an appropriate carrier, such as saline or a phosphate saline buffer. Allergen can be administered by injection subcutaneously, intramuscularly, or intraperitoneally (most humans would be treated by subcutaneous injection), by aerosol, inhaled powder, or by ingestion.

Therapy or desensitization with the modified allergens can be used in combination with other therapies, such as allergen-non-specific anti-IgE antibodies to deplete the patient of allergen-specific IgE antibodies (Boulet, et al. (1997) 155:1835-1840; Fahy, et al. (1997) American J Respir. Crit. Care Med. 155:1828-1834; Demoly, P. and Bousquet (1997) J Am J Resp. Crit. Care Med. 155:1825-1827), or by the pan specific anti-allergy therapy described in U. S. Serial No. 08/090,375 filed June 4, 1998, by M. Caplan and H. Sosin. Therapy with the modified allergen can also be administered in combination with an adjuvant such as IL 12, IL 16, IL 18, Ifn-ξ.

The nucleotide molecule encoding the modified allergen can also be administered directly to the patient, for example, in a suitable expression vector such as a plasmid, which is injected directly into the muscle or dermis, or through administration of genetically engineered cells.

In general, effective dosages will be in the picogram to milligram range, more typically microgram to milligram. Treatment will typically be between twice/weekly and once a month, continuing for up to three to five years, although this is highly dependent on the individual patient response.

The modified allergen can also be used as a diagnostic to characterize the patient's allergies, using techniques such as those described in the examples.

### EXAMPLES

Peanut allergy is one of the most common and serious of the immediate hypersensitivity reactions to foods in terms of persistence and severity of reaction. Unlike the clinical symptoms of many other food allergies, the reactions to peanuts are rarely outgrown, therefore, most diagnosed children will have the disease for a lifetime (Sampson, H.A., and Burks, A.W. (1996) Annu. Rev. Nutr. 16, 161-77; Bock, S.A. (1985) J. Pediatr. 107, 676-680). The majority of cases of fatal food-induced anaphylaxis involve ingestion of peanuts (Sampson et al., (1992) NEJM 327, 380-384; Kaminogawa, S. (1996) Biosci. Biotech. Biochem. 60, 1749-1756). The only effective therapeutic option currently available for the prevention of a peanut hypersensitivity reaction is food avoidance. Unfortunately, for a ubiquitous food such as a peanut, the possibility of an inadvertent ingestion is great.

The examples described below demonstrate identification, modification, and assessment of allergenicity of the major peanut allergens, Ara h 1, Ara h 2, and Ara h 3. Detailed experimental procedures are included for Example 1. These same procedures were used for Examples 2-5. The nucleotide sequences of Ara h 1, Ara h 2, and Ara h 3, are shown in SEQ ID NOs. 1, 3, and 5, respectively. The amino acid sequences of Ara h 1, Ara h 2, and Ara h 3 are shown in SEQ ID NOs. 2, 4, and 6 respectively.

### Example 1: Identification of linear IgE binding epitopes.

Due to the significance of the allergic reaction and the widening use of peanuts as protein extenders in processed foods, there is increasing interest in defining the allergenic proteins and exploring ways to decrease the risk to the peanut-sensitive individual. Various studies over the last several years have identified the major allergens in peanuts as belonging to different families of seed storage proteins (Burks, et al. (1997) Eur. J. Biochem. 245, 334-339; Stanley, et al. (1997) Arch. Biochem. Biophys. 342, 244-253). The major peanut allergens Ara h 1, Ara h 2, and Ara h 3 belong to the vicilin, conglutin and glycinin families of seed storage proteins, respectively. These allergens are abundant proteins found in peanuts and are recognized by serum IgE from greater than 95% of peanut sensitive individuals, indicating that they are the major allergens involved in the clinical etiology of this disease (Burks, et al. (1995) J. Clinical Invest., 96, 1715-1721). The genes encoding Ara h 1 (SEQ ID NO. 1), Ara h 2 (SEQ ID NO. 3), and Ara h 3 (SEQ ID NO. 5) and the proteins encoded by these genes (SEQ ID NO. 2, 4, 6) have been isolated and characterized. The following studies were conducted to identify the IgE epitopes of these allergens recognized by a population of peanut hypersensitive patients and a means for modifying their affinity for IgE.

### Experimental Procedures

***Serum IgE*.** Serum from 15 patients with documented peanut hypersensitivity reactions (mean age, 25 yrs) was used to determine relative binding affinities between wild type and mutant Ara h 1 synthesized epitopes. The patients had either a positive double-blind, placebo-controlled, food challenge or a convincing history of peanut anaphylaxis (laryngeal edema, severe wheezing, and/or hypotension; Burks, et al. (1988) J. Pediatr. 113, 447-451). At least 5 ml of venous blood was drawn from each patient, allowed to clot, and serum was collected. A serum pool from 12 to 15 patients was made by mixing equal aliquots of serum IgE from each patient. The pools were then used in immunoblot analysis.

***Peptide synthesis*.** Individual peptides were synthesized on a derivatized cellulose membrane using 9-fluorenyllmethoxycarbonyl (Fmoc) amino acid active esters according to the manufacturer's instructions (Genosys Biotechnologies, Woodlands, Texas; Fields, G.B. and Noble, R.L. (1990) Int. J. Peptide Protein Res. 35, 161-214). Fmoc-amino acids (N-terminal blocked) with protected side chains were coupled in the presence of 1-methyl-2-pyrrolidone to a derivatized cellulose membrane. Following washing with dimethylformamide (DMF), unreacted terminal amino groups were blocked from further reactions by acetylation with acetic anhydride. The N-terminal Fmoc blocking group was then removed by reaction with 20% piperidine and 80% DMF, v/v. The membrane was washed in DMF followed by methanol, the next reactive Fmoc-amino acid was then coupled as before, and the sequence of reactions was repeated with the next amino acid. When peptide synthesis was complete, the side chains were deprotected with a mixture of dichloromethane (DCM), triflouroacetic acid, and triisobutylsilane (1.0:1.0:0.5), followed by successive washes in DCM, DMF, and methanol. Peptides synthesis reactions were monitored by bromophenol blue color reactions during certain steps of synthesis. Cellulose derivitised membranes and Fmoc-amino acids were supplied by Genosys Biotechnologies. All other chemical were purchased from Aldrich Chemical Company, Inc. (Milwaukee, WI) or Fluka (Buchs, Switzerland). Membranes were either probed immediately or stored at -20°C until needed.

***IgE binding assays*.** Cellulose membranes containing synthesized peptides were washed 3 times in Tris-buffered saline (TBS; 136 mM NaCl, 2.7 mM KCI, and 50 mM trizma base pH 8.0) for 10 min at room temperature (RT) and then incubated overnight in blocking buffer: [TBS, 0.05% Tween^{™} 20; concentrated membrane blocking buffer supplied by Genosys; and sucrose (0.0:1.0:0.5)]. The membrane was then incubated in pooled sera diluted in 1:5 in 20 mM Tris-Cl pH7.5, 150 mM NaCl, and 1% bovine serum albumin overnight at 4°C. Primary antibody was detected with ¹²⁵I-labeled equine anti-human IgE (Kallestad, Chaska, MN).

***Quantitation of IgE binding*.** Relative amounts of IgE binding to individual peptides were determined by a Bio-Rad (Hercules, CA) model GS-700 imaging laser densitometer and quantitated with Bio-Rad molecular analyst software. A background area was scanned and subtracted from the obtained values. Following quantitation, wild type intensities were normalized to a value of one and the mutants were calculated as percentages relative to the wild type.

***Synthesis and purification of recombinant Ara h 2 protein*.** cDNA encoding Ara h 2 was placed in the pET-24b expression vector. The pET-24 expression vector places a 6 x histidine tag at the carboxyl end of the inserted protein. The histidine tag allows the recombinant protein to be purified by affinity purification on a nickel column (HisBind resin). Recombinant Ara h 2 was expressed and purified according to the instructions of the pET system manual. Briefly, expression of the recombinant Ara h 2 was induced in 200 ml cultures of strain BL21(DE3) E. coli with 1 mM IPTG at mid log phase. Cultures were allowed to continue for an additional 3 hours at 36°C. Cells were harvested by centrifugation at 2000 x g for 15 minutes and then lysed in denaturing binding buffer (6 M urea, 5 mM imidazole, 0.5 M NaCl, 20 mM Tris-HCl, pH 7.9). Lysates were cleared by centrifugation at 39,000 x g for 20 minutes followed by filtration though 0.45 micron filters. The cleared lysate was applied to a 10 ml column of HisBind resin, washed with imidazole wash buffer (20 mM imidazole, 6 M urea, 0.5 M NaCl, 20 mM Tris-HCl, pH 7.9). The recombinant Ara h 2 was then released from the column using elution buffer (1 M imidazole, 0.5 M NaCl, 20 mM Tris-HCl, pH 7.9). The elution buffer was replaced with phosphate buffered saline by dialysis. The purification of recombinant Ara h 2 was followed by SDS PAGE and immunoblots. Peanut specific serum IgE was used as a primary antibody.

***Skin prick tests.*** The ability of purified native and recombinant Ara h 2 to elicit the IgE mediated degranulation of mast cells was evaluated using prick skin tests in a peanut allergic individual. An individual meeting the criteria for peanut allergy (convincing history or positive double blind placebo controlled food challenge) and a non-allergic control were selected for the testing. Purified native and recombinant Ara h 2 and whole peanut extract (Greer Laboratories, Lenoir, N.C.) were tested. Twenty microliters of the test solution were applied to the forearm of the volunteer and the skin beneath pricked with a sterile needle. Testing was started at the lowest concentration (less than or equal to 1 mg/ml) and increased ten fold each round to the highest concentration or until a positive reaction was observed. Mean diameters of the wheal and erythema were measured and compared to the negative saline control. A positive reaction was defined as a wheal 3mm larger then the negative control. Histamine was used as the positive control.

### Results

*Identification of the linear IgE-binding epitopes of Ara h 1, Ara h 2 and Ara h 3 allergens.* Epitope mapping was performed on the Ara h 1, Ara h 2 and Ara h 3 allergens by synthesizing each of these proteins in 15 amino acid long overlapping peptides that were offset from each other by 8 amino acids. The peptides were then probed with a pool of serum IgE from 15 patients with documented peanut hypersensitivity. This analysis resulted in multiple IgE binding regions being identified for each allergen. The exact position of each IgE binding epitope was then determined by re-synthesizing these IgE reactive regions as 10 or 15 amino acid long peptides that were offset from each other by two amino acids. These peptides were probed with the same pool of serum IgE from peanut sensitive patients as used before. An example of this procedure for each of the peanut allergens is shown in Figures 1-3. Figure 1 shows amino acid residues 82-133 ofAra h 1, containing peptides 4, 5, 6, and 7, as identified in Table 1. Figure 2 shows amino acid residues 55-76 of Ara h 2, containing peptides 6 and 7, as shown in Table 2. Figure 3 shows amino acid residues 299-321 of Ara h 3, containing peptide 4 as identified in Table 3. This analysis revealed that there were 23 linear IgE binding epitopes on Ara h 1, 10 epitopes on Ara h 2, and 4 epitopes on Ara h 3.

In an effort to determine which, if any, of the epitopes were recognized by the majority of patients with peanut hypersensitivity, each set of epitopes identified for the peanut allergens were synthesized and then probed individually with serum IgE from 10 different patients. All of the patient sera tested recognized multiple epitopes.

Table 1 shows the amino acid sequence and position of each epitope within the Ara h 1 protein of all 23 IgE binding epitopes mapped to this molecule. Table 2 shows the amino acid sequence and position of each epitope within the Ara h 2 protein of all 10 IgE binding epitopes mapped to this molecule. Table 3 shows the amino acid sequence and position of each epitope within the Ara h 3 protein of all 4 IgE binding epitopes mapped to this molecule.

Four epitopes of the Ara h 1 allergen (peptides 1, 3, 4, 17 of Table 1), three epitopes of the Ara h 2 allergen (peptides 3, 6, 7 of Table 2), and 1 epitope of the Ara h 3 allergen (peptide 2 of Table 3) were immunodominant.

**Table 1. Ara h I IgE Binding Epitopes**

| **EPITOPE** | **AA SEQUENCE** | **POSITION** |
|---|---|---|
| 1 | AKSSPYQKKT | 25-34 |
| 2 | QEPDDLKQKA | 48-57 |
| 3 | LEYDPRLVYD | 65-74 |
| 4 | GERTRGRQPG | 89-98 |
| 5 | PGDYDDDRRQ | 97-106 |
| 6 | PRREEGGRWG | 107-116 |
| 7 | REREEDWRQP | 123-132 |
| 8 | EDWRRPSHQQ | 134-143 |
| 9 | QPRKIRPEGR | 143-152 |
| 10 | TPGQFEDFFP | 294-303 |
| 11 | SYLQEFSRNT | 311-320 |
| 12 | FNAEFNEIRR | 325-334 |
| 13 | EQEERGQRRW | 344-353 |
| 14 | DITNPINLRE | 393-402 |
| 15 | NNFGKLFEVK | 409-418 |
| 16 | GTGNLELVAV | 461-470 |
| 17 | RRYTARLKEG | 498-507 |
| 18 | ELHLLGFGIN | 525-534 |
| 19 | HRIFLAGDKD | 539-548 |
| 20 | IDQIEKQAKD | 551-560 |
| 21 | KDLAFPGSGE | 559-568 |
| 22 | KESHFVSARP | 578-587 |
| 23 | PEKESPEKED | 597-606 |

The underlined portions of each peptide are the smallest IgE binding sequences as determined by this analysis. All of these sequences can be found in SEQ ID NO 2.

**Table 2. Ara h 2 IgE Binding Epitopes**

| **EPITOPE** | **AA SEQUENCE** | **POSITION** |
|---|---|---|
| 1 | HASARQQWEL | 15-24 |
| 2 | QWELQGDRRC | 21-30 |
| 3 | DRRCQSQLER | 27-36 |
| 4 | LRPCEQHLMQ | 39-48 |
| 5 | KIQRDEDSYE | 49-58 |
| 6 | YERDPYSPSQ | 57-66 |
| 7 | SQDPYSPSPY | 65-74 |
| 8 | DRLQGRQQEQ | 115-124 |
| 9 | KRELRNLPQQ | 127-136 |
| 10 | QRCDLDVESG | 143-152 |

The underlined portions of each peptide are the smallest IgE binding sequences as determined by this analysis. All of these sequences can be found in SEQ ID NO 4.

**Table 3. Ara h 3 IgE Binding Epitopes**

| **EPITOPE** | **AA SEQUENCE** | **POSITION** |
|---|---|---|
| 1 | IETWNPNNQFFFCAG | 33-47 |
| 2 | GNIFSGFTPEFLEQA | 240-254 |
| 3 | VTVRGGLRILSPDRK | 279-293 |
| 4 | DEDEYEYDEEDRRRG | 303-317 |

The underlined portions of each peptide are the smallest IgE binding sequences as determined by this analysis. All of these sequences can be found in SEQ ID NO 6.

### Example 2 : Modification of peanut allergens to decrease allergenicity.

The major linear IgE binding epitopes of the peanut allergens were mapped using overlapping peptides synthesized on an activated cellulose membrane and pooled serum IgE from 15 peanut sensitive patients, as described in Example 1. The size of the epitopes ranged from six to fifteen amino acids in length. The amino acids essential to IgE binding in each of the epitopes were determined by synthesizing duplicate peptides with single amino acid changes at each position. These peptides were then probed with pooled serum IgE from 15 patients with peanut hypersensitivity to determine if the changes affected peanut-specific IgE binding. For example, epitope 9 in Table 1 was synthesized with an alanine or methionine residue substituted for one of the amino acids and probed. The following amino acids were substituted (first letter is the one-letter amino acid code for the residue normally at the position, the residue number, followed by the amino acid that was substituted for this residue; the numbers indicate the position of each residue in the Ara h 1 protein, SEQ ID NO. 2): Q143A, P144A; R145A; K146A; I147A; R148A; P149A; E150A; G151A; R152A; Q143M; P144M; R145M; K146M; I147M; R148M; P149M; E150M; G151M; R152M. The immunoblot strip containing the wild-type and mutated peptides of epitope 9 showed that binding of pooled serum IgE to individual peptides was dramatically reduced when either alanine or methionine was substituted for each of the amino acids at positions 144, 145, and 147-150 of Ara h 1 shown in SEQ ID NO. 2. Changes at positions 144, 145, 147, and 148 of Ara h 1 shown in SEQ ID NO. 2 had the most dramatic effect when methionine was substituted for the wild-type amino acid, resulting in less than 1% of peanut specific IgE binding to these peptides. In contrast, the substitution of an alanine for arginine at position 152 of Ara h 1 shown in SEQ ID NO. 2 resulted in increased IgE binding. The remaining Ara h 1 epitopes, and the Ara h 2 and Ara h 3 epitopes, were tested in the same manner and the intensity of IgE binding to each spot was determined as a percentage of IgE binding to the wild-type peptide. Any amino acid substitution that resulted in less than 1% of IgE binding when compared to the wild type peptide was noted and is indicated in Tables 4-6. Table 4 shows the amino acids that were determined to be critical to IgE binding in each of the Ara h 1 epitopes. Table 5 shows the amino acids that were determined to be critical to IgE binding in each of the Ara h 2 epitopes. Table 6 shows the amino acids that were determined to be critical to IgE binding in each of the Ara h 3 epitopes.

This analysis indicated that each epitope could be mutated to a non-IgE binding-peptide by the substitution of a single amino acid residue.

The results discussed above for Ara h 1, Ara h 2, and Ara h 3 demonstrate that once an IgE binding site has been identified, it is possible to reduce IgE binding to this site by altering a single amino acid of the epitope. The observation that alteration of a single amino acid leads to the loss of IgE binding in a population of peanut-sensitive individuals is significant because it suggests that while each patient may display a polyclonal IgE reaction to a particular allergen, IgE from different patients that recognize the same epitope must interact with that epitope in a similar fashion. Besides finding that many epitopes contained more than one residue critical for IgE binding, it was also determined that more than one residue type (ala or met) could be substituted at certain positions in an epitope with similar results. This allows for the design of a hypoallergenic protein that would be effective at blunting allergic reactions for a population of peanut sensitive individuals. Furthermore, the creation of a plant producing a peanut where the IgE binding epitopes of the major allergens have been removed should prevent the development of peanut hypersensitivity in individuals genetically predisposed to this food allergy.

**Table 4: Amino Acids Critical to IgE Binding of Ara h 1**

| **EPITOPE** | **AA SEQUENCE** | **POSITION** |
|---|---|---|
| 1 | AKS**SPY**Q**K**KT | 25-34 |
| 2 | QEP**DDL**KQKA | 48-57 |
| 3 | LE**YDP**RL**VY**D | 65-74 |
| 4 | GE**R**TR**GRQ**PG | 89-98 |
| 5 | PGDYDD**D**RRQ | 97-106 |
| 6 | PRREE**G**GRWG | 107-116 |
| 7 | REREED**W**R**Q**P | 123-132 |
| 8 | EDW**RRP**SHQQ | 134-143 |
| 9 | Q**PR**K**IR**PEGR | 143-152 |
| 10 | T**P**GQ**F**ED**FF**P | 294-303 |
| 11 | S**YL**Q**EF**SRNT | 311-320 |
| 12 | **F**NAE**F**NEIRR | 325-334 |
| 13 | EQEER**G**QRRW | 344-353 |
| 14 | DIT**NPI**N**L**RE | 393-402 |
| 15 | NNFGK**LF**EVK | 409-418 |
| 17 | **RRY**TARLKEG | 498-507 |
| 18 | EL**HL**L**GFG**IN | 525-534 |
| 19 | HRIFLAGD**K**D | 539-548 |
| 20 | IDQ**I**EKQ**A**K**D** | 551-560 |
| 21 | KDLA**FPG**SGE | 559-568 |
| 22 | KESHFV**S**ARP | 578-587 |

| | | |
|---|---|---|
| *Note*. The Ara h 1 IgE binding epitopes are indicated as the single letter amino acid code. The position of each peptide with respect to the Ara h 1 protein is indicated in the right hand column. The amino acids that, when altered, lead to loss of IgE binding are shown as the bold, underlined residues. Epitopes 16 and 23 were not included in this study because they were recognized by a single patient who was no longer available to the study. All of these sequences can be found in SEQ ID NO 2. | | |

**Table 5. Amino Acids Critical to IgE Binding of Ara h 2**

| **EPITOPE** | **AA SEQUENCE** | **POSITION** |
|---|---|---|
| 1 | HASAR**Q**Q**W**EL | 15-24 |
| 2 | Q**W**E**L**Q**G**DRRC | 21-30 |
| 3 | D**RR**C**Q**SQL**ER** | 27-36 |
| 4 | **L**R**P**CE**QH**LMQ | 39-48 |
| 5 | **K**IQ**RD**E**D**SYE | 49-58 |
| 6 | YER**DPY**SPSQ | 57-66 |
| 7 | SQ**DPY**SPSPY | 65-74 |
| 8 | DRL**QGR**QQEQ | 115-124 |
| 9 | **KR**E**L**RN**L**PQQ | 127-136 |
| 10 | QRC**DL**D**VE**SG | 143-152 |

| | | |
|---|---|---|
| *Note*. The Ara h 2 IgE binding epitopes are indicated as the single letter amino acid code. The position of each peptide with respect to the Ara h 2 protein is indicated in the right hand column. The amino acids that, when altered, lead to loss of IgE binding are shown as the bold, underlined residues. All of these sequences can be found in SEQ ID NO 4. | | |

**Table 6. Amino Acids Critical to IgE-Binding of Ara h 3.**

| **EPITOPE** | **AA SEQUENCE** | **POSITION** |
|---|---|---|
| 1 | IETWN**PN**NQEFECAG | 33-47 |
| 2 | GNI**F**SG**F**TPE**FL**EQA | 240-254 |
| 3 | VTVRGG**L**R**IL**S**P**DRK | 279-293 |
| 4 | DEDEY**EYDE**E**DR**RRG | 303-317 |

| | | |
|---|---|---|
| *Note*. The Ara h 3 IgE binding epitopes are indicated as the single letter amino acid code. The position of each peptide with respect to the Ara h 3 protein is indicated in the right hand column. The amino acids that, when altered, lead to loss of IgE binding are shown as the bold, underlined All of these sequences can be found in SEQ ID NO 6. | | |

### Example 3: A Modified Ara h 2 Protein Binds less IgE But Similar Amounts of IgG.

In order to determine the effect of changes to multiple epitopes within the context of the intact allergen, four epitopes (including the three immunodominant epitopes) of the Ara h 2 allergen were mutagenized and the protein produced recombinantly. The amino acids at position 20, 31, 60, and 67 of the Ara h 2 protein (shown in SEQ ID NO. 4) were changed to alanine by mutagenizing the gene encoding this protein by standard techniques. These residues are located in epitopes 1, 3, 6, and 7 and represent amino acids critical to IgE binding that were determined in Example 2. The modified and wild-type versions of this protein were produced and immunoblot analysis performed using serum from peanut sensitive patients. These results showed that the modified version of this allergen bound significantly less IgE than the wild type version of these recombinant proteins (Figure 4) but bound similar amounts of IgG.

### Example 4: A modified Ara h 2 protein retains the ability to stimulate T-cells to proliferate.

The modified recombinant Ara h 2 protein described in Example 3 was used in T-cell proliferation assays to determine if it retained the ability to activate T cells from peanut sensitive individuals. Proliferation assays were performed on T-cell lines grown in short-term culture developed from six peanut sensitive patients. T-cells lines were stimulated with either 50 µg of crude peanut extract, 10 µg of native Ara h 2, 10 µg of recombinant wild-type Ara h2, or 10 µg of modified recombinant Ara h 2 protein and the amount of 3H-thymidine determined for each cell line. Results were expressed as the average stimulation index (SI) which reflected the fold increase in 3H-thymidine incorporation exhibited by cells challenged with allergen when compared with media treated controls (Figure 5).

### Example 5: A Modified Ara h 2 Protein Elicits a Smaller Wheal and Flare in Skin Prick Tests of a Peanut Sensitive Individual.

The modified recombinant Ara h 2 protein described in Example 3 and the wild type version of this recombinant protein were used in a skin prick test of a peanut sensitive individual. Ten micrograms of these proteins were applied separately to the forearm of a peanut sensitive individual, the skin pricked with a sterile needle, and 10 minutes later any wheal and flare that developed was measured. The wheal and flare produced by the wild-type Ara h 2 protein (8 mm X 7 mm) was approximately twice as large as that produced by the modified Ara h 2 protein (4 mm X 3mm). A control subject (no peanut hypersensitivity) tested with the same proteins had no visible wheal and flare but, as expected, gave positive results when challenged with histamine. In addition, the test subject gave no positive results when tested with PBS alone. These results indicate that an allergen with only 40% of its IgE binding epitopes modified (4/10) can give measurable reduction in reactivity in an *in vivo* test of a peanut sensitive patient.

These same techniques can be used with the other known peanut allergens, Ara h 1 (SEQ ID NO 1 and 2), Ara h 3 (SEQ ID NO. 5 and 6), or any other allergen.

## Claims

1. A transgenic plant expressing a modified peanut allergen whose amino acid sequence is substantially identical to that of a natural peanut allergen of SEQ ID NO:4 except that at least one amino acid has been modified by substitution in at least three IgE epitopes so that IgE binding to the modified peanut allergen is reduced as compared with IgE binding to the natural peanut allergen, wherein the modified peanut allergen activates T cells and said IgE epitopes are ones that are recognised when the natural peanut allergen is contacted with a pool of sera IgE from individuals that are allergic to the natural peanut allergen, wherein the modified peanut allergen comprises a modification in each immunodominant epitope of SEQ ID NO:4, which immunodominant epitopes are found at amino acids 27 to 36 of SEQ ID NO:4, amino acids 57-66 of SEQ ID NO:4 and amino acids 65-74 of SEQ ID NO:4, and wherein the modification, by substitution, is of as little as a single amino acid of the bold underlined residues in each of the immunodominant epitopes of SEQ ID NO:4:
D**RR**C**Q**SQL**ER**
YER**DPY**SPSQ
SQ**DPY**SPSPY.

2. The transgenic plant of claim 1 wherein at least one amino acid has been modified in 10 IgE epitopes of SEQ ID NO:4.

3. The transgenic plant of claim 1 wherein at least one modified amino acid is located in the centre of at least one IgE epitope.

4. The transgenic plant of claim 3 wherein a hydrophobic amino acid in the centre of an IgE epitope has been substituted by a neutral or hydrophilic amino acid.

5. The transgenic plant of claim 1 wherein the modified peanut allergen binds IgG.

6. The transgenic plant of claim 1 wherein the modified peanut allergen activates a Th1-type response.

7. A transgenic plant as defined in any one of claims 1 to 6 for use in medicine.

8. Use of a transgenic plant as defined in any one of claims 1 to 6 in the manufacture of a medicament for reducing the clinical response to a natural peanut allergen in an individual.

## Patentansprüche

1. Transgene Pflanze, die ein modifiziertes Erdnussallergen exprimiert, dessen Aminosäuresequenz im Wesentlichen zu jener eines natürlichen Erdnussallergens von SEQ ID Nr. 4 identisch ist, mit der Ausnahme, dass zumindest eine Aminosäure durch Substitution in zumindest drei IgE-Epitopen modifiziert wurde, so dass die IgE-Bindung an das modifizierte Erdnussallergen im Vergleich mit einer IgE-Bindung an das natürliche Erdnussallergen verringert ist, wobei das modifizierte Erdnussallergen T-Zellen aktiviert, und wobei die IgE-Epitope welche sind, die erkannt werden, wenn das natürliche Erdnussallergen mit einem Pool von Seren-IgE von Individuen, die auf das natürliche Erdnussallergen allergisch sind, in Kontakt gebracht wird,, wobei das modifizierte Erdnussallergen eine Modifikation in jedem immundominanten Epitop von SEQ ID Nr. 4 umfasst, wobei die immundominanten Epitope an den Aminosäuren 27 bis 36 der SEQ ID. Nr. 4, Aminosäuren 57-66 von SEQ ID Nr. 4 und Aminosäuren 65-74 von SEQ ID Nr. 4 gefunden werden, und wobei die Modifikation durch Substitution nur eine einzelne Aminosäure der fetten, unterstrichenen Reste in jedem der immundominanten Epitope von SEQ ID Nr. 4 ist:
D**RR**C**Q**SQL**ER**
YER**DPY**SPSQ
SQ**DPY**SPSPY

2. Transgene Pflanze nach Anspruch 1, wobei zumindest eine Aminosäure in 10 IgE-Epitopen von SEQ ID Nr. 4 modifiziert wurde.

3. Transgene Pflanze nach Anspruch 1, wobei zumindest eine Aminosäure im Zentrum zumindest eines IgE-Epitops angeordnet ist.

4. Transgene Pflanze nach Anspruch 3, wobei eine hydrophobe Aminosäure im Zentrum eines IgE-Epitops durch eine neutrale oder hydrophile Aminosäure ersetzt wurde.

5. Transgene Pflanze nach Anspruch 1, wobei das modifizierte Erdnussallergen IgG bindet.

6. Transgene Pflanze nach Anspruch 1, wobei das modifizierte Erdnussallergen eine Th1-Typ-Antwort aktiviert.

7. Transgene Pflanze nach einem der Ansprüche 1 bis 6 zur Verwendung in der Medizin.

8. Verwendung einer transgenen Pflanze nach einem der Ansprüche 1 bis 6 bei der Herstellung eines Medikaments zum Reduzieren der klinischen Antwort auf ein natürliches Erdnussallergen bei einem Individuum.

## Revendications

1. Plante transgénique exprimant un allergène de cacahuète modifié dont la séquence d'acides aminés est sensiblement identique à celle d'un allergène de cacahuète naturel de SEQ ID No. : 4 si ce n'est qu'au moins un acide aminé a été modifié par substitution dans au moins trois épitopes d'IgE de sorte que la liaison d'IgE à l'allergène de cacahuète modifié est réduite par comparaison avec la liaison d'IgE à l'allergène de cacahuète naturel, dans laquelle l'allergène de cacahuète modifié active les cellules T et lesdits épitopes d'IgE sont ceux qui sont reconnus lorsque l'allergène de cacahuète naturel est mis en contact avec un ensemble d'IgE de sérum provenant d'individus qui sont allergiques à l'allergène de cacahuète naturel, dans laquelle l'allergène de cacahuète modifié comprend une modification dans chaque épitope immunodominant de SEQ ID No. : 4, lesquels épitopes immunodominants sont trouvés aux acides aminés 27 à 36 de SEQ ID No. : 4, les acides aminés 57 à 66 de SEQ ID No. : 4 et les acides aminés 65 à 74 de SEQ ID No. : 4, et dans laquelle la modification, par substitution, est aussi petite qu'un seul acide aminé des résidus soulignés en caractères gras dans chacun des épitopes immunodominants de SEQ ID No. : 4 :
D**RR**C**Q**SQL**ER**
YER**DPY**SPSQ
SQ**DPY**SPSPY.

2. Plante transgénique selon la revendication 1, dans laquelle au moins un acide aminé a été modifié dans 10 épitopes d'IgE de SEQ ID No. : 4.

3. Plante transgénique selon la revendication 1, dans laquelle au moins un acide aminé modifié est situé au centre d'au moins un épitope d'IgE.

4. Plante transgénique selon la revendication 3, dans laquelle un acide aminé hydrophobe au centre d'un épitope d'IgE a été substitué par un acide aminé neutre ou hydrophile.

5. Plante transgénique selon la revendication 1, dans laquelle l'allergène de cacahuète modifié lie IgG.

6. Plante transgénique selon la revendication 1, dans laquelle l'allergène de cacahuète modifié active une réponse de type Th1.

7. Plante transgénique telle que définie dans l'une quelconque des revendications 1 à 6, pour une utilisation en médecine.

8. Utilisation d'une plante transgénique telle que définie dans l'une quelconque des revendications 1 à 6 dans la fabrication d'un médicament pour réduire la réponse clinique à un allergène de cacahuète naturel chez un individu.


### SEQUENCE LISTING

<110> University of Arkansas
   Mt. Sinai School of Medicine of the City University of New York Sosin, Howard
<120> Methods and Reagents for Decreasing Clinical Reaction to Allergy
<130> HS102
<140>
   <141>
<150> 09/141220
   <151> 1998-08-27
<150> 60/074590
   <151> 1998-02-13
<150> 60/074624
   <151> 1998-02-13
<150> 60/074633
   <151> 1998-02-13
<150> 60/073283
   <151> 1998-01-29
<160> 6
<170> PatentIn Ver. 2.0
<210> 1
   <211> 1930
   <212> DNA
   <213> Peanut
<400> 1
   aataatcata tatattcatc aatcatctat ataagtagta gcaggagcaa tgagagggag 60
   ggtttctcca ctgatgctgt tgctagggat ccttgtcctg gcttcagttt ctgcaacgca 120
   tgccaagtca tcaccttacc agaagaaaac agagaacccc tgcgcccaga ggtgcctcca 180
   gagttgtcaa caggaaccgg atgacttgaa gcaaaaggca tgcgagtctc gctgcaccaa 240
   gctcgagtat gatcctcgtt gtgtctatga tcctcgagga cacactggca ccaccaacca 300
   acgttcccct ccaggggagc ggacacgtgg ccgccaaccc ggagactacg atgatgaccg 360
   ccgtcaaccc cgaagagagg aaggaggccg atggggacca gctggaccga gggagcgtga 420
   aagagaagaa gactggagac aaccaagaga agattggagg cgaccaagtc atcagcagcc 480
   acggaaaata aggcccgaag gaagagaagg agaacaagag tggggaacac caggtagcca 540
   tgtgagggaa gaaacatctc ggaacaaccc tttctacttc ccgtcaaggc ggtttagcac 600
   ccgctacggg aaccaaaacg gtaggatccg ggtcctgcag aggtttgacc aaaggtcaag 660
   gcagtttcag aatctccaga atcaccgtat tgtgcagatc gaggccaaac ctaacactct 720
   tgttcttccc aagcacgctg atgctgataa catccttgtt atccagcaag ggcaagccac 780
   cgtgaccgta gcaaatggca ataacagaaa gagctttaat cttgacgagg gccatgcact 840
   cagaatccca tccggtttca tttcctacat cttgaaccgc catgacaacc agaacctcag 900
   agtagctaaa atctccatgc ccgttaacac acccggccag tttgaggatt tcttcccggc 960
   gagcagccga gaccaatcat cctacttgca gggcttcagc aggaatacgt tggaggccgc 1020
   cttcaatgcg gaattcaatg agatacggag ggtgctgtta gaagagaatg caggaggtga 1080
   gcaagaggag agagggcaga ggcgatggag tactcggagt agtgagaaca atgaaggagt 1140
   gatagtcaaa gtgtcaaagg agcacgttga agaacttact aagcacgcta aatccgtctc 1200
   aaagaaaggc tccgaagaag agggagatat caccaaccca atcaacttga gagaaggcga 1260
   gcccgatctt tctaacaact ttgggaagtt atttgaggtg aagccagaca agaagaaccc 1320
   ccagcttcag gacctggaca tgatgctcac ctgtgtagag atcaaagaag gagctttgat 1380
   gctcccacac ttcaactcaa aggccatggt tatcgtcgtc gtcaacaaag gaactggaaa 1440
   ccttgaactc gtggctgtaa gaaaagagca acaacagagg ggacggcggg aagaagagga 1500
   ggacgaagac gaagaagagg agggaagtaa cagagaggtg cgtaggtaca cagcgaggtt 1560
   gaaggaaggc gatgtgttca tcatgccagc agctcatcca gtagccatca acgcttcctc 1620
   cgaactccat ctgcttggct tcggtatcaa cgctgaaaac aaccacagaa tcttccttgc 1680
   aggtgataag gacaatgtga tagaccagat agagaagcaa gcgaaggatt tagcattccc 1740
   tgggtcgggt gaacaagttg agaagctcat caaaaaccag aaggaatctc actttgtgag 1800
   tgctcgtcct caatctcaat ctcaatctcc gtcgtctcct gagaaagagt ctcctgagaa 1860
   agaggatcaa gaggaggaaa accaaggagg gaagggtcca ctcctttcaa ttttgaaggc 1920
   ttttaactga                                                        1930
<210> 2
   <211> 626
   <212> PRT
   <213> Peanut
<220>
   <221> PEPTIDE
   <222> (25)..(34)
   <223> peptide 1
<220>
   <221> PEPTIDE
   <222> (48)..(57)
   <223> peptide 2
<220>
   <221> PEPTIDE
   <222> (65)..(74)
   <223> peptide 3
<220>
   <221> PEPTIDE
   <222> (89)..(98)
   <223> peptide 4
<220>
   <221> PEPTIDE
   <222> (97)..(106)
   <223> peptide 5
<220>
   <221> PEPTIDE
   <222> (107)..(116)
   <223> peptide 6
<220>
   <221> PEPTIDE
   <222> (123)..(132)
   <223> peptide 7
<220>
   <221> PEPTIDE .
   <222> (134)..(143)
   <223> peptide 8
<220>
   <221> PEPTIDE
   <222> (143)..(152)
   <223> peptide 9
<220>
   <221> PEPTIDE
   <222> (294)..(303)
   <223> peptide 10
<220>
   <221> PEPTIDE
   <222> (311)..(320)
   <223> peptide 11
<220>
   <221> PEPTIDE
   <222> (325)..(334)
   <223> peptide 12
<220>
   <221> PEPTIDE
   <222> (344)..(353)
   <223> peptide 13
<220>
   <221> PEPTIDE
   <222> (393)..(402)
   <223> peptide 14
<220>
   <221> PEPTIDE
   <222> (409)..(418)
   <223> peptide 15
<220>
   <221> PEPTIDE
   <222> (461)..(470)
   <223> peptide 16
<220>
   <221> PEPTIDE
   <222> (498)..(507)
   <223> peptide 17
<220>
   <221> PEPTIDE
   <222> (525)..(534)
   <223> peptide 18
<220>
   <221> PEPTIDE
   <222> (539)..(548)
   <223> peptide 19
<220>
   <221> PEPTIDE
   <222> (551)..(560)
   <223> peptide 20
<220>
   <221> PEPTIDE
   <222> (559)..(568)
   <223> peptide 21
<220>
   <221> PEPTIDE
   <222> (578)..(587)
   <223> peptide 22
<220>
   <221> PEPTIDE
   <222> (597)..(606)
   <223> peptide 23
<400> 2
   Met Arg Gly Arg Val Ser Pro Leu Met Leu Leu Leu Gly Ile Leu Val
    1               5                  10                  15
   Leu Ala Ser Val Ser Ala Thr His Ala Lys Ser Ser Pro Tyr Gln Lys
               20                  25                  30
   Lys Thr Glu Asn Pro Cys Ala Gln Arg Cys Leu Gln Ser Cys Gln Gln
           35                  40                  45
   Glu Pro Asp Asp Leu Lys Gln Lys Ala Cys Glu Ser Arg Cys Thr Lys
       50                  55                  60
   Leu Glu Tyr Asp Pro Arg Cys Val Tyr Asp Pro Arg Gly His Thr Gly
   65                  70                  75                  80
   Thr Thr Asn Gln Arg Ser Pro Pro Gly Glu Arg Thr Arg Gly Arg Gln
                   85                  90                  95
   Pro Gly Asp Tyr Asp Asp Asp Arg Arg Gln Pro Arg Arg Glu Glu Gly
               100                 105                 110
   Gly Arg Trp Gly Pro Ala Gly Pro Arg Glu Arg Glu Arg Glu Glu Asp
           115                 120                 125
   Trp Arg Gln Pro Arg Glu Asp Trp Arg Arg Pro Ser His Gln Gln Pro
       130                 135                 140
   Arg Lys Ile Arg Pro Glu Gly Arg Glu Gly Glu Gln Glu Trp Gly Thr
   145                 150                 155                 160
   Pro Gly Ser His Val Arg Glu Glu Thr Ser Arg Asn Asn Pro Phe Tyr
                   165                 170                 175
   Phe Pro Ser Arg Arg Phe Ser Thr Arg Tyr Gly Asn Gln Asn Gly Arg
               180                 185                 190
   Ile Arg Val Leu Gln Arg Phe Asp Gln Arg Ser Arg Gln Phe Gln Asn
           195                 200                 205
   Leu Gln Asn His Arg Ile Val Gln Ile Glu Ala Lys Pro Asn Thr Leu
       210                 215                 220
   Val Leu Pro Lys His Ala Asp Ala Asp Asn Ile Leu Val Ile Gln Gln
   225                 230                 235                 240
   Gly Gln Ala Thr Val Thr Val Ala Asn Gly Asn Asn Arg Lys Ser Phe
                   245                 250                 255
   Asn Leu Asp Glu Gly His Ala Leu Arg Ile Pro Ser Gly Phe Ile Ser
               260                 265                 270
   Tyr Ile Leu Asn Arg His Asp Asn Gln Asn Leu Arg Val Ala Lys Ile
           275                 280                 285
   Ser Met Pro Val Asn Thr Pro Gly Gln Phe Glu Asp Phe Phe Pro Ala
       290                 295                 300
   Ser Ser Arg Asp Gln Ser Ser Tyr Leu Gln Gly Phe Ser Arg Asn Thr
   305                 310                 315                 320
   Leu Glu Ala Ala Phe Asn Ala Glu Phe Asn Glu Ile Arg Arg Val Leu
                   325                 330                 335
   Leu Glu Glu Asn Ala Gly Gly Glu Gln Glu Glu Arg Gly Gln Arg Arg
               340                 345                 350
   Trp Ser Thr Arg Ser Ser Glu Asn Asn Glu Gly Val Ile Val Lys Val
           355                 360                 365
   Ser Lys Glu His Val Glu Glu Leu Thr Lys His Ala Lys Ser Val Ser
       370                 375                 380
   Lys Lys Gly Ser Glu Glu Glu Gly Asp Ile Thr Asn Pro Ile Asn Leu
   385                 390                 395                 400
   Arg Glu Gly Glu Pro Asp Leu Ser Asn Asn Phe Gly Lys Leu Phe Glu
                   405                 410                 415
   Val Lys Pro Asp Lys Lys Asn Pro Gln Leu Gln Asp Leu Asp Met Met
               420                 425                 430
   Leu Thr Cys Val Glu Ile Lys Glu Gly Ala Leu Met Leu Pro His Phe
           435                 440                 445
   Asn Ser Lys Ala Met Val Ile Val Val Val Asn Lys Gly Thr Gly Asn
       450                 455                 460
   Leu Glu Leu Val Ala Val Arg Lys Glu Gln Gln Gln Arg Gly Arg Arg
   465                 470                 475                 480
   Glu Glu Glu Glu Asp Glu Asp Glu Glu Glu Glu Gly Ser Asn Arg Glu
                   485                 490                 495
   Val Arg Arg Tyr Thr Ala Arg Leu Lys Glu Gly Asp Val Phe Ile Met
               500                 505                 510
   Pro Ala Ala His Pro Val Ala Ile Asn Ala Ser Ser Glu Leu His Leu
           515                 520                 525
   Leu Gly Phe Gly Ile Asn Ala Glu Asn Asn His Arg Ile Phe Leu Ala
       530                 535                 540
   Gly Asp Lys Asp Asn Val Ile Asp Gln Ile Glu Lys Gln Ala Lys Asp
   545                 550                 555                 560
   Leu Ala Phe Pro Gly Ser Gly Glu Gln Val Glu Lys Leu Ile Lys Asn
                   565                 570                 575
   Gln Lys Glu Ser His Phe Val Ser Ala Arg Pro Gln Ser Gln Ser Gln
               580                 585                 590
   Ser Pro Ser Ser Pro Glu Lys Glu Ser Pro Glu Lys Glu Asp Gln Glu
           595                 600                 605
   Glu Glu Asn Gln Gly Gly Lys Gly Pro Leu Leu Ser Ile Leu Lys Ala
       610                 615                 620
   Phe Asn
   625
<210> 3
   <211> 474
   <212> DNA
   <213> Peanut
<400> 3
   ctcaccatac tagtagccct cgcccttttc ctcctcgctg cccacgcatc tgcgaggcag 60
   cagtgggaac tccaaggaga cagaagatgc cagagccagc tcgagagggc gaacctgagg 120
   ccctgcgagc aacatctcat gcagaagatc caacgtgacg aggattcata tgaacgggac 180
   ccgtacagcc ctagtcagga tccgtacagc cctagtccat atgatcggag aggcgctgga 240
   tcctctcagc accaagagag gtgttgcaat gagctgaacg agtttgagaa caaccaaagg 300
   tgcatgtgcg aggcattgca acagatcatg gagaaccaga gcgataggtt gcaggggagg 360
   caacaggagc aacagttcaa gagggagctc aggaacttgc ctcaacagtg cggccttagg 420
   gcaccacagc gttgcgactt ggacgtcgaa agtggcggca gagacagata ctaa       474
<210> 4
   <211> 157
   <212> PRT
   <213> Peanut
<220>
   <221> PEPTIDE
   <222> (15)..(24)
   <223> peptide 1
<220>
   <221> PEPTIDE
   <222> (21)..(30)
   <223> peptide 2
<220>
   <221> PEPTIDE
   <222> (27)..(36)
   <223> peptide 3
<220>
   <221> PEPTIDE
   <222> (39)..(48)
   <223> peptide 4
<220>
   <221> PEPTIDE
   <222> (49)..(58)
   <223> peptide 5
<220>
   <221> PEPTIDE
   <222> (57)..(66)
   <223> peptide 6
<220>
   <221> PEPTIDE
   <222> (65)..(74)
   <223> peptide 7
<220>
   <221> PEPTIDE
   <222> (115)..(124)
   <223> peptide 8
<220>
   <221> PEPTIDE
   <222> (127)..(136)
   <223> peptide 9
<220>
   <221> PEPTIDE
   <222> (143)..(152)
   <223> peptide 10
<400> 4
   Leu Thr Ile Leu Val Ala Leu Ala Leu Phe Leu Leu Ala Ala His Ala
     1               5                  10                  15
   Ser Ala Arg Gln Gln Trp Glu Leu Gln Gly Asp Arg Arg Cys Gln Ser
                20                  25                  30
   Gln Leu Glu Arg Ala Asn Leu Arg Pro Cys Glu Gln His Leu Met Gln
            35                  40                  45
   Lys Ile Gln Arg Asp Glu Asp Ser Tyr Glu Arg Asp Pro Tyr Ser Pro
        50                  55                  60
   Ser Gln Asp Pro Tyr Ser Pro Ser Pro Tyr Asp Arg Arg Gly Ala Gly
    65                  70                  75                  80
   Ser Ser Gln His Gln Glu Arg Cys Cys Asn Glu Leu Asn Glu Phe Glu
                    85                  90                  95
   Asn Asn Gln Arg Cys Met Cys Glu Ala Leu Gln Gln Ile Met Glu Asn
               100                 105                 110
   Gln Ser Asp Arg Leu Gln Gly Arg Gln Gln Glu Gln Gln Phe Lys Arg
           115                 120                 125
   Glu Leu Arg Asn Leu Pro Gln Gln Cys Gly Leu Arg Ala Pro Gln Arg
       130                 135                 140
   Cys Asp Leu Asp Val Glu Ser Gly Gly Arg Asp Arg Tyr
   145                 150                 155
<210> 5
   <211> 1524
   <212> DNA
   <213> Peanut
<400> 5
   cggcagcaac cggaggagaa cgcgtgccag ttccagcgcc tcaatgcgca gagacctgac 60
   aatcgcattg aatcagaggg cggttacatt gagacttgga accccaacaa ccaggagttc 120
   gaatgcgccg gcgtcgccct ctctcgctta gtcctccgcc gcaacgccct tcgtaggcct 180
   ttctactcca atgctcccca ggagatcttc atccagcaag gaaggggata ctttgggttg 240
   atattccctg gttgtcctag acactatgaa gagcctcaca cacaaggtcg tcgatctcag 300
   tcccaaagac caccaagacg tctccaagga gaagaccaaa gccaacagca acgagatagt 360
   caccagaagg tgcaccgttt cgatgagggt gatctcattg cagttcccac cggtgttgct 420
   ttctggctct acaacgacca cgacactgat gttgttgctg tttctcttac tgacaccaac 480
   aacaacgaca accagcttga tcagttcccc aggagattca atttggctgg gaacacggag 540
   caagagttct taaggtacca gcaacaaagc agacaaagca gacgaagaag cttaccatat 600
   agcccataca gcccgcaaag tcagcctaga caagaagagc gtgaatttag ccctcgagga 660
   cagcacagcc gcagagaacg agcaggacaa gaagaagaaa acgaaggtgg aaacatcttc 720
   agcggcttca cgccggagtt cctggaacaa gccttccagg ttgacgacag acagatagtg 780
   caaaacctaa gaggcgagac cgagagtgaa gaagagggag ccattgtgac agtgagggga 840
   ggcctcagaa tcttgagccc agatagaaag agacgtgccg acgaagaaga ggaatacgat 900
   gaagatgaat atgaatacga tgaagaggat agaaggcgtg gcaggggaag cagaggcagg 960
   gggaatggta ttgaagagac gatctgcacc gcaagtgcta aaaagaacat tggtagaaac 1020
   agatcccctg acatctacaa ccctcaagct ggttcactca aaactgccaa cgatctcaac 1080
   cttctaatac ttaggtggct tggacctagt gctgaatatg gaaatctcta caggaatgca 1140
   ttgtttgtcg ctcactacaa caccaacgca cacagcatca tatatcgatt gaggggacgg 1200
   gctcacgtgc aagtcgtgga cagcaacggc aacagagtgt acgacgagga gcttcaagag 1260
   ggtcacgtgc ttgtggtgcc acagaacttc gccgtcgctg gaaagtccca gagcgagaac 1320
   ttcgaatacg tggcattcaa gacagactca aggcccagca tagccaacct cgccggtgaa 1380
   aactccgtca tagataacct gccggaggag gtggttgcaa attcatatgg cctccaaagg 1440
   gagcaggcaa ggcagcttaa gaacaacaac cccttcaagt tcttcgttcc accgtctcag 1500
   cagtctccga gggctgtggc ttaa                                        1524
<210> 6
   <211> 510
   <212> PRT
   <213> Peanut
<220>
   <221> PEPTIDE
   <222> (33)..(47)
   <223> peptide 1
<220>
   <221> PEPTIDE
   <222> (240)..(254)
   <223> peptide 2
<220>
   <221> PEPTIDE
   <222> (279)..(293)
   <223> peptide 3
<220>
   <221> PEPTIDE
   <222> (303)..(317)
   <223> peptide 4
<400> 6
   Ile Ser Phe Arg Gln Gln Pro Glu Glu Asn Ala Cys Gln Phe Gln Arg
    1               5                  10                  15
   Leu Asn Ala Gln Arg Pro Asp Asn Arg Ile Glu Ser Glu Gly Gly Tyr
               20                  25                  30
   Ile Glu Thr Trp Asn Pro Asn Asn Gln Glu Phe Glu Cys Ala Gly Val
           35                  40                  45
   Ala Leu Ser Arg Leu Val Leu Arg Arg Asn Ala Leu Arg Arg Pro Phe
       50                  55                  60
   Tyr Ser Asn Ala Pro Gln Glu Ile Phe Ile Gln Gln Gly Arg Gly Tyr
   65 70 75 80
   Phe Gly Leu Ile Phe Pro Gly Cys Pro Arg His Tyr Glu Glu Pro His
   85 90 95
   Thr Gln Gly Arg Arg Ser Gln Ser Gln Arg Pro Pro Arg Arg Leu Gln
   100 105 110
   Gly Glu Asp Gln Ser Gln Gln Gln Arg Asp Ser His Gln Lys Val His
   115 120 125
   Arg Phe Asp Glu Gly Asp Leu Ile Ala Val Pro Thr Gly Val Ala Phe
   130 135 140
   Trp Leu Tyr Asn Asp His Asp Thr Asp Val Val Ala Val Ser Leu Thr
   145 150 155 160
   Asp Thr Asn Asn Asn Asp Asn Gln Leu Asp Gln Phe Pro Arg Arg Phe
   165 170 175
   Asn Leu Ala Gly Asn Thr Glu Gln Glu Phe Leu Arg Tyr Gln Gln Gln
   180 185 190
   Ser Arg Gln Ser Arg Arg Arg Ser Leu Pro Tyr Ser Pro Tyr Ser Pro
   195 200 205
   Gln Ser Gln Pro Arg Gln Glu Glu Arg Glu Phe Ser Pro Arg Gly Gln
   210 215 220
   His Ser Arg Arg Glu Arg Ala Gly Gln Glu Glu Glu Asn Glu Gly Gly
   225 230 235 240
   Asn Ile Phe Ser Gly Phe Thr Pro Glu Phe Leu Glu Gln Ala Phe Gln
   245 250 255
   Val Asp Asp Arg Gln Ile Val Gln Asn Leu Arg Gly Glu Thr Glu Ser
   260 265 270
   Glu Glu Glu Gly Ala Ile Val Thr Val Arg Gly Gly Leu Arg Ile Leu
   275 280 285
   Ser Pro Asp Arg Lys Arg Arg Ala Asp Glu Glu Glu Glu Tyr Asp Glu
   290 295 300
   Asp Glu Tyr Glu Tyr Asp Glu Glu Asp Arg Arg Arg Gly Arg Gly Ser
   305 310 315 320
   Arg Gly Arg Gly Asn Gly Ile Glu Glu Thr Ile Cys Thr Ala Ser Ala
   325 330 335
   Lys Lys Asn Ile Gly Arg Asn Arg Ser Pro Asp Ile Tyr Asn Pro Gln
   340 345 350
   Ala Gly Ser Leu Lys Thr Ala Asn Asp Leu Asn Leu Leu Ile Leu Arg
   355 360 365
   Trp Leu Gly Leu Ser Ala Glu Tyr Gly Asn Leu Tyr Arg Asn Ala Leu
   370 375 380
   Phe Val Ala His Tyr Asn Thr Asn Ala His Ser Ile Ile Tyr Arg Leu
   385                 390                 395                 400
   Arg Gly Arg Ala His Val Gln Val Val Asp Ser Asn Gly Asn Arg Val
                   405                 410                 415
   Tyr Asp Glu Glu Leu Gln Glu Gly His Val Leu Val Val Pro Gln Asn
               420                 425                 430
   Phe Ala Val Ala Gly Lys Ser Gln Ser Glu Asn Phe Glu Tyr Val Ala
           435                 440                 445
   Phe Lys Thr Asp Ser Arg Pro Ser Ile Ala Asn Leu Ala Gly Glu Asn
       450                 455                 460
   Ser Val Ile Asp Asn Leu Pro Glu Glu Val Val Ala Asn Ser Tyr Gly
   465                 470                 475                 480
   Leu Gln Arg Glu Gln Ala Arg Gln Leu Lys Asn Asn Asn Pro Phe Lys
                   485                 490                 495
   Phe Phe Val Pro Pro Ser Gln Gln Ser Pro Arg Ala Val Ala
               500                 505                 510